# EUROPEAN PATENT SPECIFICATION

(11) **EP 2 508 117 B1**
(45) Date of publication and mention of the grant of the patent: **01.04.2015**
(21) Application number: 11828740.8
(22) Date of filing: 07.09.2011
(51) Int. Cl.: A61B 1/00, A61B 1/01

(54) **INSERTION AID, AND ENDOSCOPE DEVICE**
EINSETZHILFE UND ENDOSKOPVORRICHTUNG
AIDE À L'INTRODUCTION ET DISPOSITIF D'ENDOSCOPE

(30) Priority: 30.09.2010 JP 2010222476
(43) Date of publication of application: 10.10.2012
(73) Proprietor: OLYMPUS MEDICAL SYSTEMS CORP., Tokyo 151-0072 (JP)
(72) Inventor: SUGIYAMA, Yuta, Tokyo 151-0072 (JP)
(74) Representative: Gunzelmann, Rainer
(86) International application number: PCT/JP2011/070407
(87) International publication number: WO 2012/043179

(56) References cited:
- JP-A- 6 292 652
- JP-A- 10 262 918
- JP-A- 55 129 029
- JP-A- 62 201 133
- JP-A- 2001 340 287
- JP-A- 2005 046 275
- JP-A- 2005 318 957
- JP-A- 2006 505 348
- JP-U- H0 255 910
- JP-U- H0 258 407
- US-A1- 2007 208 224
- US-A1- 2008 091 170

## Description

### Technical Field

The present invention relates to an insertion aid which is arranged along a longitudinal direction of an insertion portion to be inserted into a subject, and an endoscope apparatus using the same.

### Background Art

As is generally known, an endoscope which is widely used in medical fields has an elongated insertion portion to be inserted into a subject. As an aid for improving the insertability when inserting the insertion portion of such an endoscope into a winding duct such as a sigmoid colon, insertion aids such as a so-called overtubes are known.

As such type of insertion aid, for example, Japanese Patent Application Laid-Open Publication No. 2006-505302 discloses an overtube which is configured to include a thermo-softening polymer layer with a wire embedded therein. This polymer layer is adapted to be softened by resistance heating of the wire by passage of an electric current, and softening of the polymer layer will turn the overtube into a state in which it has a small shape retaining force and is flexible. On the other hand, when the passage of electric current to the wire is stopped, the polymer layer will be hardened so that the overtube turns in a state in which it has a large shape retaining force and is less prone to be curved and deformed. Such a property is utilized, in the technique of Patent Literature 1, to selectively harden the insertion aid (overtube), thereby reducing the expansion of an organ caused by the advancement of the insertion portion of the endoscope.

Incidentally, during insertion into a subject or the like, for example, at a proximal end side of an insertion portion or an insertion aid, a moment of a force applied to a distal end side works. Since a moment is expressed as a product of force and distance as is generally known, fundamentally the longer the insertion path, the larger the moment that acts on a proximal end side of an insertion portion or the like. Accordingly, in order to prevent buckling or the like caused by such a moment and achieve high insertability, it is desirable to set an insertion aid to have a high flexural rigidity.

On the other hand, if an insertion aid is set to have a high flexural rigidity, it is concerned that interference or the like of an insertion aid against an intestinal wall is caused more than is necessary particularly in the case of the sigmoid colon or the like where the insertion path is complexedly curved, which results in the subject being forcedly applied with a burden.

Document US 2008/091170 A1 discloses a shape-transferring cannula that creates a custom-contoured access port for insertion and removal of diagnostic, surgical, or interventional instruments to and from a site within the body to which the physician does not have line-of-sight access. The shape-transferring cannula is constructed of a normally-rigid core and sheath elements which, in proper sequence, become flexible when energized and re-stiffen when they return to an un-energized state.

The present invention was made in view of the above-mentioned circumstances, and an objective of the present invention is to provide an insertion aid and an endoscope apparatus capable of achieving high insertability without applying a burden on a subject even in cases such as when the insertion path is long or complexedly curved.

### Disclosure of Invention

Means for Solving the Problem

An insertion aid according to the present invention comprises the features of claim 1.

Moreover, an endoscope apparatus according to the present invention comprises the features of claim 4.

### Brief Description of the Drawings

Fig. 1 is a schematic block diagram of an endoscope apparatus relating to a first embodiment of the present invention.
Fig. 2 is a perspective view of an endoscope equipped with an insertion aid relating to the first embodiment of the present invention.
Fig. 3 is a principal-part cross-sectional view of an insertion portion of the endoscope relating to the first embodiment of the present invention.
Fig. 4 is a principal-part cross-sectional view of the insertion aid relating to the first embodiment of the present invention.
Fig. 5 is a V-V cross-sectional view of Fig. 4 relating to the first embodiment of the present invention.
Fig. 6 is a VI-VI cross-sectional view of Fig. 4 relating to the first embodiment of the present invention.
Fig. 7 is an explanatory diagram regarding a reactive force relating to the first embodiment of the present invention.
Fig. 8 is an explanatory diagram showing the states of the distal end portion and the aid relating to the first embodiment of the present invention when they are inserted into the sigmoid colon.
Fig. 9 is an explanatory diagram showing the states of the distal end portion and the aid relating to the first embodiment of the present invention when they are inserted into the sigmoid colon.
Fig. 10 is an explanatory diagram showing the states of the distal end portion and the aid relating to the first embodiment of the present invention when they are inserted into the sigmoid colon.
Fig. 11 is an explanatory diagram showing the states of the distal end portion and the aid relating to the first embodiment of the present invention when they are inserted into the sigmoid colon.
Fig. 12 is an explanatory diagram showing the states of the distal end portion and the aid relating to the first embodiment of the present invention when they are inserted into the sigmoid colon.
Fig. 13 is an explanatory diagram showing the states of the distal end portion and the aid relating to the first embodiment of the present invention when they are inserted into the sigmoid colon.
Fig. 14 is an explanatory diagram showing the states of the distal end portion and the aid relating to the first embodiment of the present invention when they are inserted into the sigmoid colon.
Fig. 15 is a principal-part cross-sectional view showing a variant of the insertion aid relating to the first embodiment of the present invention.
Fig. 16 is a principal-part cross-sectional view showing a variant of the insertion aid relating to the first embodiment of the present invention.
Fig. 17 is a principal-part cross-sectional view showing variants of the insertion portion and the insertion aid relating to the first embodiment of the present invention.
Fig. 18 is a principal-part cross-sectional view showing variants of the insertion portion and the insertion aid relating to the first embodiment of the present invention.
Fig. 19 is a principal-part cross-sectional view of an insertion aid relating to a second embodiment of the present invention.
Fig. 20 is a XX-XX cross-sectional view of Fig. 19 relating to the second embodiment of the present invention.
Fig. 21 is a XXI-XXI cross-sectional view of Fig. 19 relating to the second embodiment of the present invention.
Fig. 22 is a cross-sectional view showing a principal part of the insertion portion with the insertion aid being inserted thereinto relating to the second embodiment of the present invention.
Fig. 23 is a principal-part cross-sectional view showing variants of the insertion portion and the insertion aid relating to the second embodiment of the present invention.
Fig. 24 is a principal-part cross-sectional view showing variants of the insertion portion and the insertion aid relating to the second embodiment of the present invention.

### Best Mode for Carrying Out the Invention

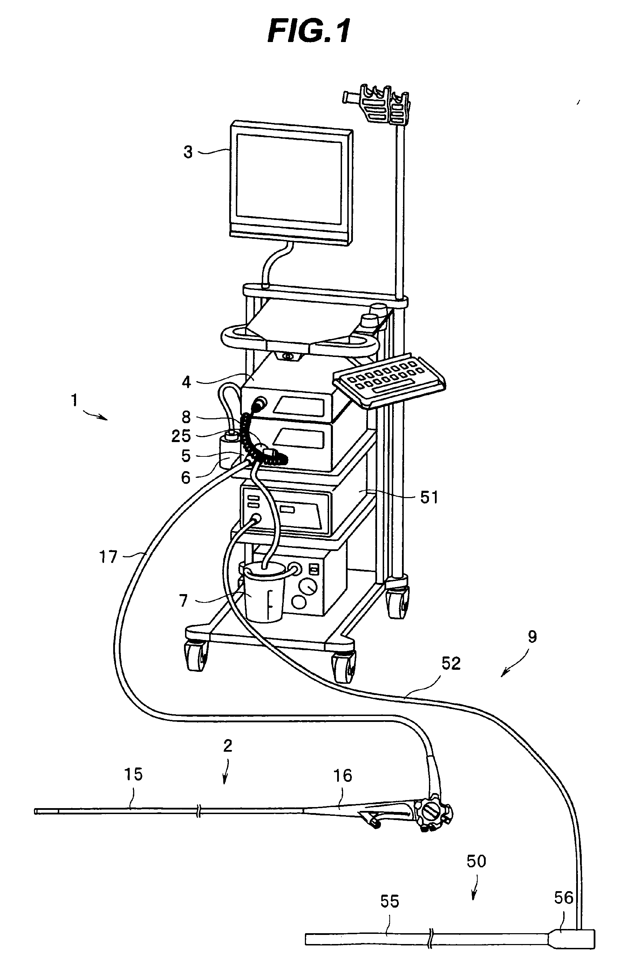

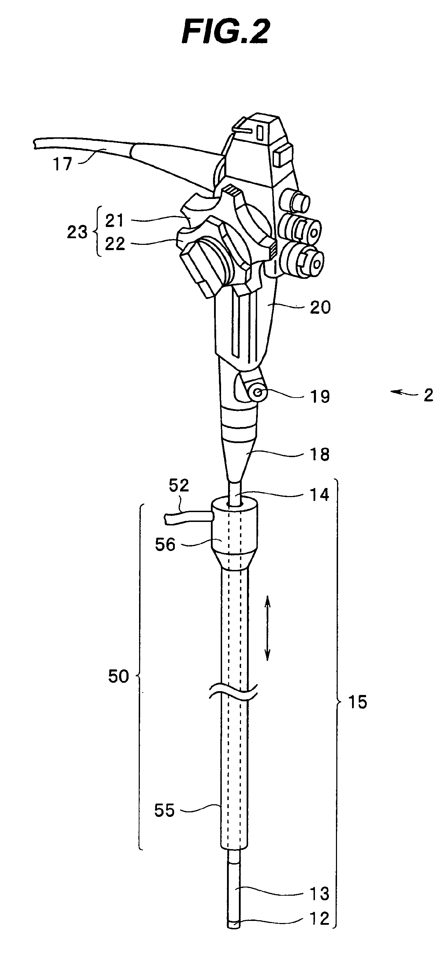

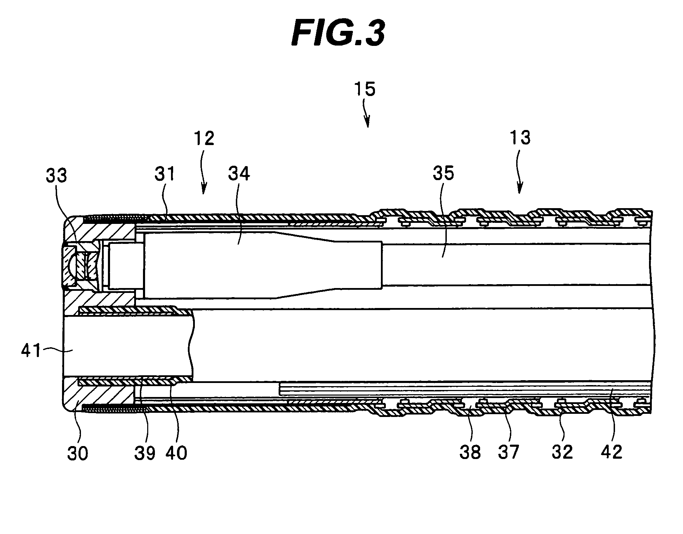

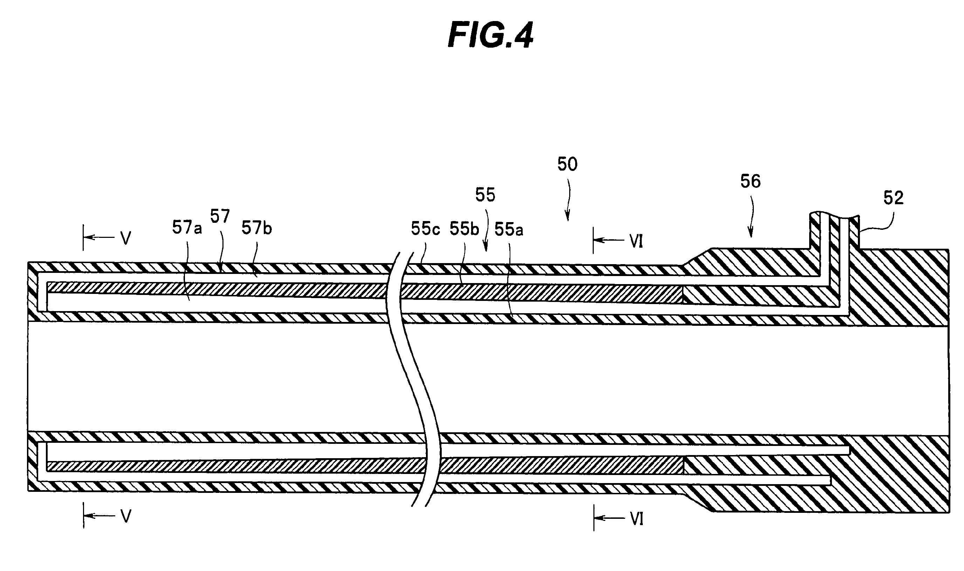

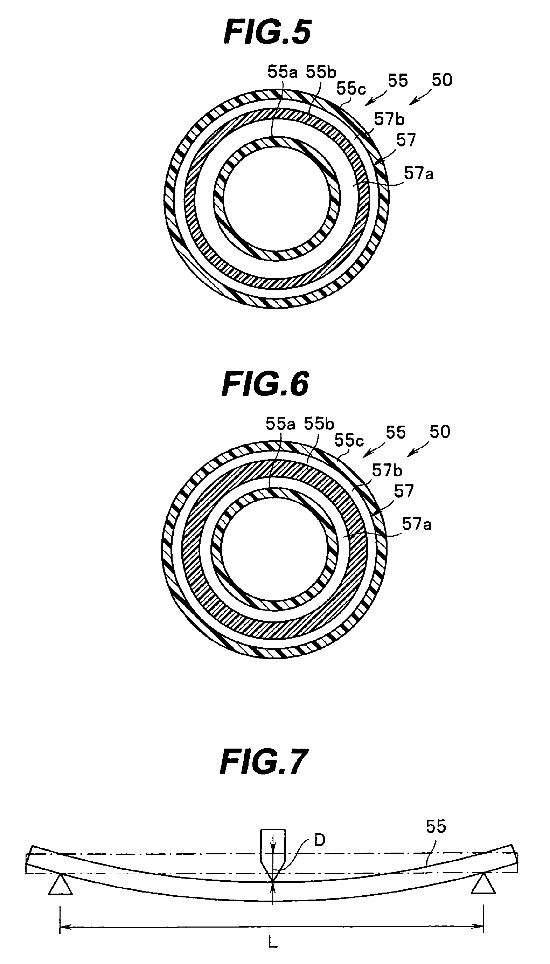

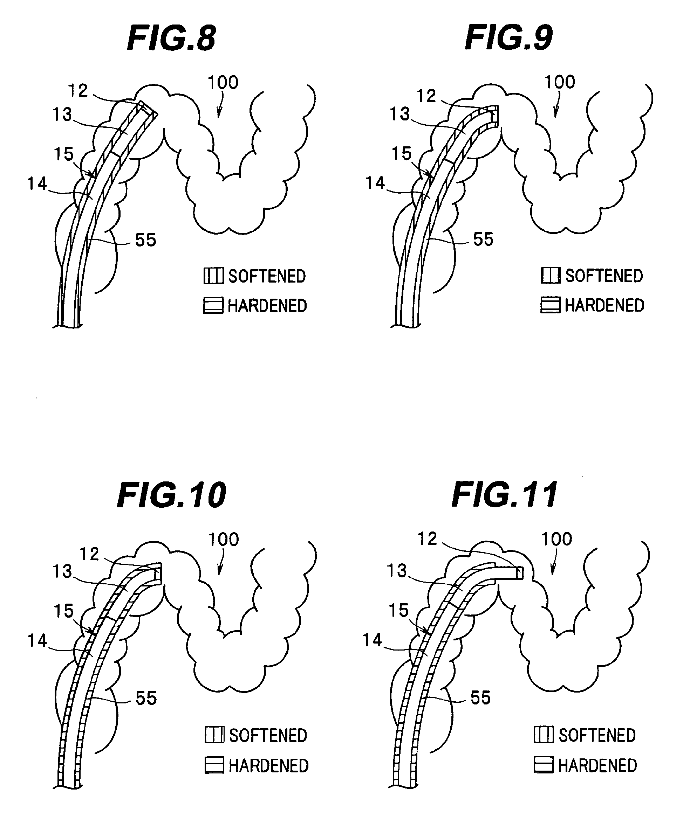

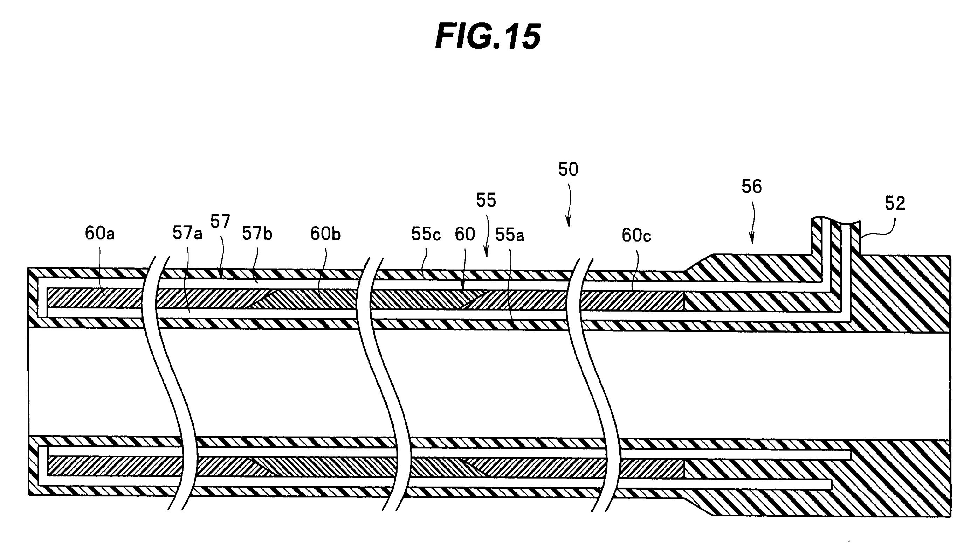

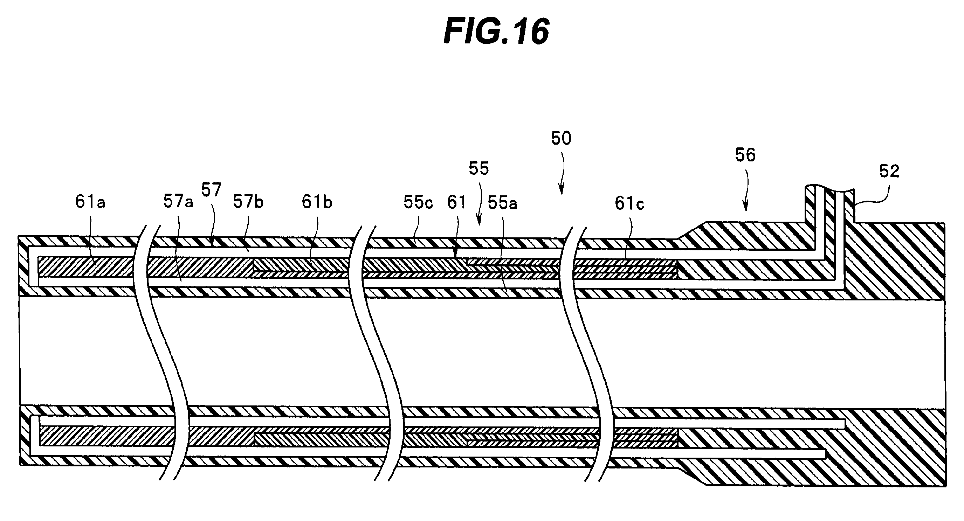

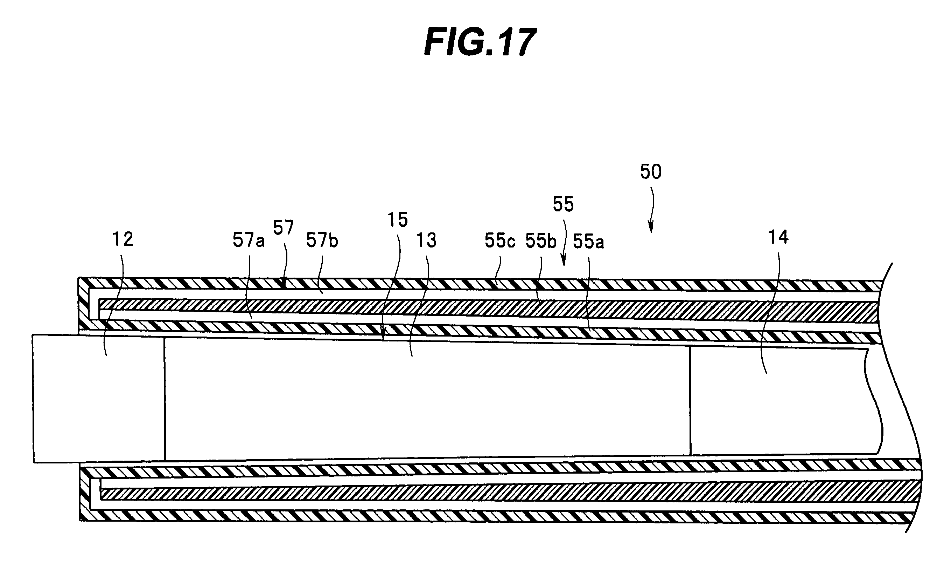

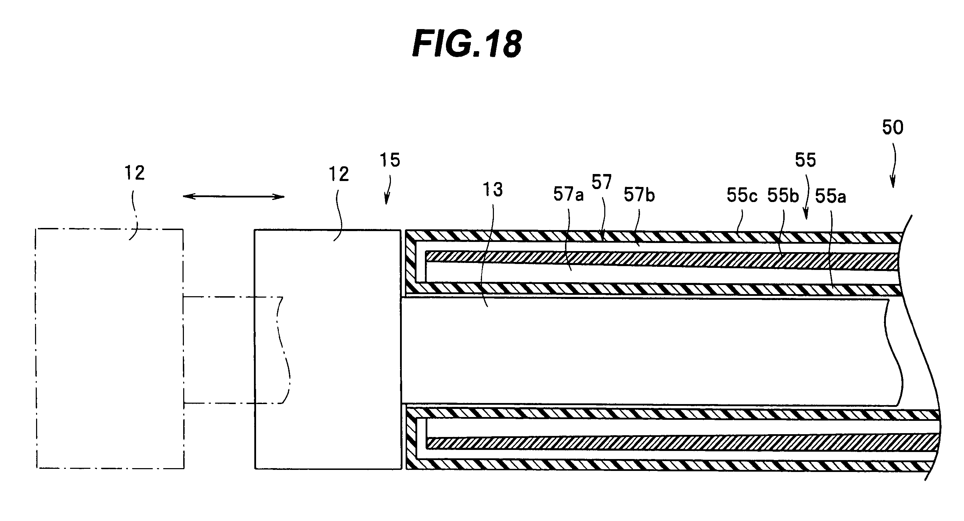

Hereafter, embodiments of the present invention will be described with reference to the drawings. Fig. 1 to Fig. 18 relate to a first embodiment of the present invention, in which Fig. 1 is a schematic block diagram of an endoscope apparatus, Fig. 2 is a perspective view of an endoscope mounted with an insertion aid, Fig. 3 is a principal-part cross-sectional view of an insertion portion of the endoscope, Fig. 4 is a principal-part cross-sectional view of the insertion aid, Fig. 5 is a V-V sectional view of Fig. 4, Fig. 6 is a VI-VI sectional view of Fig. 4, Fig. 7 is an explanatory diagram regarding a reactive force. Figs. 8 to 14 are explanatory diagrams showing the states of the distal end portion and the aid when they are inserted into the sigmoid colon, Figs. 15 and 16 are principal-part cross-sectional views showing variants of the insertion aid, and Figs. 17 and 18 are principal-part cross-sectional views showing variants of the insertion portion and the insertion aid.

A principal part of the endoscope apparatus 1 shown in Fig. 1 is configured to include, for example, an endoscope 2, a monitor 3, a video processor 4, a light source apparatus 5, an air and water supply tank 6, a suction pump 7, a signal cable 8, and an insertion aid system 9.

For example, as shown in Fig. 2, the endoscope 2 is configured to include a long and elongated insertion portion 15, an operation section 16, and a universal cord 17 incorporating various electrical signal transmission cables, light guides and the like. Further, the insertion portion 15 is configured to include a distal end portion 12, a bending portion 13, and a flexible pipe portion 14, in that order from the distal end side.

Here, for example, as shown in Fig. 3, the distal end portion 12 includes a distal-end rigid portion 30 which is a metal block, and a metal rigid pipe 31 which is fitted to the rear (the base portion side) of the distal-end rigid portion 30, and these distal-end rigid portion 30 and the rigid pipe 31 are integrally covered by an outer skin 32 thereby constituting a principal part.

A lens unit 33 constituting an objective optical system is held in the distal-end rigid portion 30. An image pickup unit 34 incorporating an image pickup device (not shown) and the like is arranged in the rear of the lens unit 33 in the optical axis direction, and an electrical signal transmission cable 35 which is incorporated in the universal cord 17 extends from the image pickup unit 34.

Further, a plurality of bending pieces 37 are arranged in one row in the bending portion 13. Among these bending pieces 37, the bending piece 37 located at the foremost position is fixedly inserted into the rear end of the rigid pipe 31, and each bending piece 37 that follows thereafter is pivotally coupled with an adjacent bending piece 37 via a pivot member 38, respectively.

Further, an opening is formed at the distal end of the distal-end rigid portion 30, and a tube-connecting pipe 39 is fixedly inserted into the opening. A channel tube 40 is connected to an outer circumferential portion of the tube-connecting pipe 39, and the channel tube 40 extends along the longitudinal direction in the insertion portion 15. As a result of this, an insertion channel 41 into which a treatment instrument or the like can be inserted is formed in the insertion portion 15.

Moreover, for example, a shape retaining member 42 made up of multiple wires is arranged to extend along the longitudinal direction in the insertion portion 15. As the wire that constitutes the shape retaining member 42, a kind which more remarkably exhibits plastic properties rather than elastic properties in a normal usage range (deformation and force range) is selected. As a result of this, the shape retaining member 42 undergoes plastic deformation when a passive bending of the insertion portion 15 due to an external force, or an active bending of the insertion portion 15 due to bending operation by the surgeon occurs, thereby retaining a bent shape of the insertion portion 15 acting against the restoring force and the like of the outer skin 32. It is noted that as the shape retaining member 42, for example, a well-known interlock tube (flexible tube) or the like may be used in place of wires and the like.

As shown in Fig. 2, the operation section 16 is configured to include a bend preventing portion 18 which is connected to the proximal end side of the flexible pipe portion 14 of the insertion portion 15, a channel insertion portion 19 in communication with the insertion channel 41 in the insertion portion 15, and an operation section body 20.

A bending operation knob 23 for performing bending operation of the bending portion 13 of the insertion portion 15 is pivotally arranged on the operation section body 20, and switches for various endoscope functions and the like are provided thereon as well. It is noted that the bending operation knob 23 includes a UD bending operation knob 21 for performing bending operation of the bending portion 13 in the up and down direction, and an RL bending operation knob 22 for performing bending operation of the bending portion 13 in the left and right direction, and these knobs are arranged in a superposed fashion.

The universal cord 17 is configured to extend from the operation section 16, and to have an endoscope connector 25, which is detachably attachable to the light source apparatus 5, at its extension end (see Fig. 1).

The video processor 4 is, for example, electrically connected with the connector 25 of the endoscope 2 via the signal cable 8 to control the operation of the image pickup unit 34 via the signal cable 8, and performs the processing of the image signal picked up at the image pickup unit 34. It is noted that an image signal processed by the video processor 4 is displayed on the monitor 3 as an endoscope image.

The light source apparatus 5 supplies illumination light to a known light guide which is not shown and provided in the endoscope 2. That is, a light guide is arranged in the universal cord 17, the operation section 16, and the insertion portion 15 of the endoscope 2 of the present embodiment, and the light source apparatus 5 supplies illumination light to the distal end portion 12 via the light guide.

The air and water supply tank 6 is connected to the light source apparatus 5 via a tube, and supplies gas or liquid to a known air and water supply duct which is not shown and provided in the endoscope 2.

The suction pump 7 is, for example, connected to the connector 25 of the endoscope 2 via a tube, and is used when suctioning liquids or tissues in the intestinal tract via a known suction duct which is not shown and provided in the endoscope 2.

As shown in Figs. 1 and 2, the insertion aid system 9 includes an insertion aid 50 which is arranged to be relatively movable in the longitudinal direction along the insertion portion 15 of the endoscope 2, and a control apparatus 51 which controls the insertion aid 50.

As shown in Figs. 1, 2, and 4, the insertion aid 50 of the present embodiment is made up of a hollow overtube which is disposed at the outer circumference of the insertion portion 15. To be specific, the insertion aid 50 is configured to include a guide portion 55 which is long and takes on a substantially cylindrical shape, and a hollow grip portion 56 which is arranged in connection with the base portion of the guide portion 55, so that the insertion portion 15 of the endoscope 2 is insertable into the interior of the guide portion 55 and the hollow grip portion 56.

As shown in Figs. 4 to 6, a principal part of the guide portion 55 is configured such that first to third tubes 55a to 55c which extend in the longitudinal direction of the guide portion 55 are arranged on concentric circles.

Among these first to third tubes 55a to 55c, the first tube 55a located at the inner most circumference and the third tube 55c located at the outer most circumference are made up of tubes which are made of resin and have flexibility. It is noted that the inner diameter of the first tube 55a is set to be slightly larger than the outer diameter of the insertion portion 15 of the endoscope 2, so that the guide portion 55 permits relative movement in the longitudinal direction of the insertion portion 15 which is inserted into the first tube 55a.

The second tube 55b is made up of, for example, a tube which utilizes a strength variable member whose strength changes according to its temperature. In the present embodiment, as the strength variable member for example, a thermosoftening polymer which softens on the high-temperatures side and hardens at the low-temperatures side can be used. In this case, as the strength variable member, for example, it is desirable to use a thermosoftening polymer whose boundary temperature to start hardening (glass transition temperature) is in a range of about 25°C to 35°C. It is noted that the temperature range of the boundary temperature described above is close to the body temperature of a human, and in a lower temperature range than the body temperature of a human, and using a thermosoftening polymer of such a temperature range will facilitate the temperature control and the like when changing the strength within a subject.

The inner diameter of the second tube 55b is set to be larger than the outer diameter of the first tube 55a, and the outer diameter of the second tube 55b is set to be smaller than the inner diameter of the third tube 55c (see Figs. 4 to 6). As a result of this, an air gap 57a is formed between the inner circumferential face of the second tube 55b and the outer circumferential face of the first tube 55a, and an air gap 57b is formed between the outer circumferential face of the second tube 55b and the inner circumferential face of the third tube 55c. Then, these air gaps 57a and 57b which are formed at the inner and outer circumferences of the second tube 55b are brought into communication with each other at the distal end side of the second tube 55b so that a series of flow path 57 is formed inside the guide portion 55.

Each end portion of the flow path 57 is connected to the control apparatus 51 via a fluid inlet/outlet duct 52 which extends from the grip portion 56. As a result of this, for example, the control apparatus 51 is enabled to recirculate the control water whose temperature is adjusted to a predetermined temperature as a control fluid, in the flow path 57, so that the heating or cooling of the second tube 55b is controlled through the control fluid that flows in the flow path 57. Then, when the entire second tube 55b is softened by the heating through the control fluid, the guide portion 55 turns into a first state in which it has a small shape retaining force and is flexible. On the other hand, when the entire second tube 55b is hardened by the cooling through the control fluid, the guide portion 55 turns into a second state in which it has a large shape retaining force and its flexibility is limited. In this way, the guide portion 55 of the present embodiment is configured to be able to make a state change between the first state in which the shape retaining force is small and the second state in which the shape retaining force is large through the temperature control of the second tube 55b.

Here, in the present invention, the shape retaining force of the guide portion 55 refers to the degree of difficulty to deform (to bend) the current shape of the guide portion 55. Although, in general, such shape retaining force is collectively judged based on various physical quantities and the like, the shape retaining force of each portion on the guide portion 55 is judged, particularly, based on the flexural rigidity. That is, in the present embodiment, it is judged that the shape retaining force is higher as the reactive force (flexural rigidity) generated at a three-point bending test becomes larger, in which for example, as shown in Fig. 7, the guide portion 55 is placed bridging over a pair of support bases which are spaced apart at a prescribed spacing L (for example, L = 150 mm), and the bridging center of the guide portion 55 is deflected by a prescribed width D (for example, D = 50 mm). Although description will be omitted, in the present embodiment, the shape retaining force of the insertion portion 15 is also judged in a similar manner.

In the present embodiment, the flexural rigidity of the guide portion 55 is prescribed, for example, based on the relationship with the flexural rigidity of the insertion portion 15 of the endoscope 2. That is, the flexural rigidity of the guide portion 55 is prescribed so as to be relatively lower than the flexural rigidity of the insertion portion 15 when the guide portion 55 is in the first state, and so as to be relatively higher than the flexural rigidity of the insertion portion 15 when in the second state. In this case, it is particularly preferable that the flexural rigidity of the guide portion 55 when in the second state is not less than three times as large as the flexural rigidity of the insertion portion 15. Moreover, it is preferable that the flexural rigidity of the guide portion 55 when in the second state is not less than five times as large as the flexural rigidity when in the first state.

In addition, a gradient is prescribed for the flexural rigidity of the guide portion 55 itself such that the flexural rigidity of the proximal end side is relatively larger than the flexural rigidity of the distal end side. Such gradient of flexural rigidity is realized, for example, as shown in Figs. 4 to 6, by providing a gradient in the wall thickness in the second tube 55b such that the wall thickness of the proximal end side is relatively larger than the wall thickness of the distal end side. That is, by making up the second tube 55b with a tapered pipe having a gradient in wall thickness, a gradient of flexural rigidity is prescribed for the guide portion 55 such that in either of the first state or the second state, the flexural rigidity of the proximal end side is relatively larger than the flexural rigidity of the distal end side.

Next, as an example of the procedure to insert the insertion portion 15 into a subject using the insertion aid system 9 of such configuration, the procedure when inserting the insertion portion 15 into the sigmoid colon will be described with reference to Figs. 8 to 14.

First, the surgeon makes the second tube 55b soften through the operation of the control apparatus 51 to set the state of the guide portion 55 to the first state in which it has flexibility. Then, the surgeon makes the guide portion 55 advance integrally with the insertion portion 15 to guide it to the proximity of the mouth of the sigmoid colon 100 (see Fig. 8). At this moment, since the flexural rigidity of the guide portion 55 in the first state is prescribed such that the flexural rigidity of the proximal end side is relatively higher than the flexural rigidity of the distal end side, the flexibility of the distal end sides of the insertion portion 15 and the guide portion 55 is sufficiently secured, and moreover even when a force from the intestinal wall or the like is applied to the distal end side of the insertion portion 15 or the like, a buckling caused by a moment due to the force will be prevented. That is, by providing a gradient in the flexural rigidity of the guide portion 55 when it is in the first state, it is made possible to secure a good insertability and to prevent buckling or the like of the guide portion 55 and the insertion portion 15 at the same time without setting the flexural rigidity of the guide portion 55 to be needlessly high over the entire area thereof.

Next, the surgeon makes the bending portion 13 bend in the advancing direction along the curving direction of the sigmoid colon 100 through the operation of the bending operation knob 23 (see Fig. 9).

Thereafter, the surgeon makes the second tube 55b harden through the operation of the control apparatus 51, thereby changing the state of the guide portion 55 to the second state in which its flexibility is limited. As a result of this, the distal end side of the guide portion 55 is held in such a shape as being bent along the curving direction of the sigmoid colon 100 (see Fig. 10).

Thereafter, the surgeon relatively moves the insertion portion 15 forward with respect to the guide portion 55 so that only the distal end side of the insertion portion 15 advances into the sigmoid colon 100 (see Fig. 11). At this moment, since the flexural rigidity of the guide portion 55 which is in the second state is prescribed to be relatively higher than (for example, not less than three times) the flexural rigidity of the insertion portion 15, even if a force when pushing out the insertion portion 15 to the distal end side of the guide portion 55 which is retained in a bent state is applied thereto, the distal end side of the guide portion 55 will not be deformed by the force so that the bent shape of the guide portion 55 is maintained. Therefore, the distal end side (the bending portion 13 and the like) of the insertion portion 15 is appropriately guided to a desired advancing direction in the sigmoid colon 100 along the bent shape of the guide portion 55. Further, since the insertion portion 15 has a shape retaining capability due to the shape retaining member 42, the distal end side of the insertion portion 15 is retained in a state of being oriented toward the desired advancing direction even after being pushed out from the guide portion 55. In addition, since the flexural rigidity of the guide portion 55 in the second state is prescribed such that the flexural rigidity of the proximal end side is relatively higher than the flexural rigidity of the distal end side, even when a force from the insertion portion 15 is applied to the distal end side of the guide portion 55 which is in a bent state, it is possible to prevent buckling or the like of the guide portion 55 caused by a moment due to the force. That is, by providing a gradient in the flexural rigidity of the guide portion 55 when in the second state, it is made possible to prevent buckling or the like of the guide portion 55 without setting the flexural rigidity of the guide portion 55 to be needlessly high over the entire area thereof.

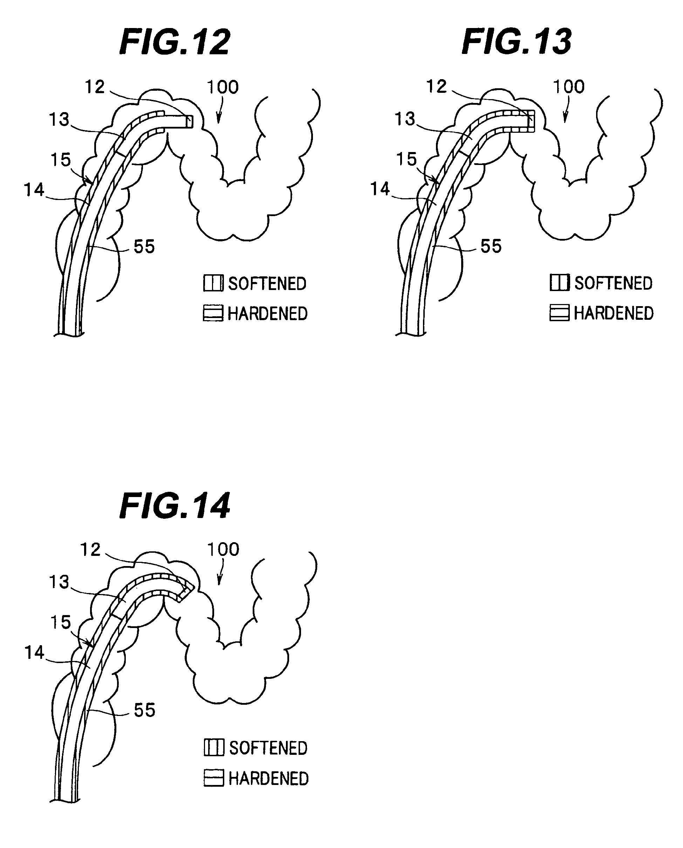

Thereafter, the surgeon makes the second tube 55b soften through the operation to the control apparatus 51 to change the state of the guide portion 55 to the second state in which it has flexibility (see Fig. 12).

Then, the surgeon relatively moves the guide portion 55 forward with respect to the insertion portion 15. This causes the distal end side of the guide portion 55 to advance into the sigmoid colon 100 along the shape of the distal end side of the insertion portion 15 (see Fig. 13). At this moment, since the flexural rigidity of the guide portion 55 in the first state is relatively lower than the flexural rigidity of the insertion portion 15, and moreover the insertion portion 15 has a shape retaining capability due to the shape retaining member 42, the distal end side of the guide portion 55 is appropriately guided to a desired advancing direction in the sigmoid colon 100 along the shape of the insertion portion 15.

Thereafter, the surgeon makes the bending portion 13 bend in the advancing direction along the curving direction of the sigmoid colon 100 through the operation of the bending operation knob 23 as in the procedure described in Fig. 9 (see Fig. 11). Thereafter, by successively repeating the procedure shown after Fig. 10, the insertion portion 15 is inserted into a deep portion of the sigmoid colon 100 along the shape thereof.

According to the embodiment as described above, by providing a gradient in the flexural rigidity of the guide portion 55 such that the flexural rigidity of the proximal end side is relatively higher than the flexural rigidity of the distal end side, it is possible to realize a high insertability without imposing a burden on a subject even when the insertion path is long, or curved in a complex shape, and the like.

That is, by providing a gradient in the flexural rigidity of the guide portion 55 such that the flexural rigidity of the base portion side, to which a relatively large moment acts, is relatively higher than the flexural rigidity of the distal end side, it is possible to appropriately prevent buckling or the like of the insertion portion 15 and the guide portion 55 thereby realizing a good insertability without setting the flexural rigidity of the guide portion 55 to be needlessly high over the entire area thereof. Further, by providing a gradient in the flexural rigidity in this way, it is possible to mitigate the insertion resistance particularly at the distal end side, thereby realizing further improvement of the insertability, and reducing the burden imposed on a subject.

Here, as the configuration to provide a gradient in the flexural rigidity of the guide portion 55, a configuration in which multiple kinds of thermosoftening polymers having different glass transition temperatures are combined, for example, as shown in Figs. 15 and 16 can be adopted in place of the configuration in which the wall thickness of the second tube 55b and the like is varied as described above.

That is, the guide portion 55 shown in Fig. 15 is provided with a gradient in flexural rigidity by differentiating the thermosoftening polymer constituting the second tube 60 according to the position in the longitudinal direction. To be specific, the second tube 60 shown in Fig. 15 is made up by successively coupling a tube portion 60a having a glass transition temperature of 25°C, a tube portion 60b having a glass transition temperature of 30°C, and a tube portion 60c having a glass transition temperature of 35°C in this order from the distal end side. Then, in the guide portion 55 having such configuration as described above, a gradient of flexural rigidity is realized by differentiating the progress condition of hardening (or softening) of each of the tube portions 60a to 60c by the temperature adjustment of control fluid through the control apparatus 51.

Moreover, the guide portion 55 shown in Fig. 16 is provided with a gradient of flexural rigidity by configuring the second tube 61 to be a multi-layered structure made up of thermosoftening polymers having different glass transition temperatures, and differentiating the number of layers according to the position in the longitudinal direction. To be specific, the second tube 61 shown in Fig. 16 is made up by successively setting an area made up of a layer 61a having a glass transition temperature of 25°C alone, an area made up of the layer 6 1 a having a glass transition temperature of 25°C and a layer 61b of 30°C, and an area made up of the layer 61a having a glass transition temperature of 25°C, the layer 61b of 30°C, and a layer 61c of 35°C in order from the distal end side. Then, in the guide portion 55 having such configuration as described above, a gradient of flexural rigidity is realized by differentiating the progress condition of hardening (or softening) of each of the layers 61a to 61c by the temperature adjustment of control fluid through the control apparatus 51.

By the way, in the endoscope apparatus 1 of this type, it is desirable to prepare a countermeasure for enabling the insertion operation to be continued even in an accidental case that the insertion portion 15 and the guide portion 55 buckle at their midway portions while being inserted into a subject. As the countermeasure, for example, it is possible, as shown in Fig. 17, to form the outer circumferential face of the insertion portion 15 with a tapered surface in which the outer diameter of the distal end side is relatively larger than the outer diameter of the proximal end side, and form the inner circumferential surface of the guide portion 55 with a tapered surface in which the inner diameter of the distal end side is relatively larger than the inner diameter of the proximal end side.

Forming each face with such a tapered surface makes it easy to push out the insertion portion 15 relatively forwardly with respect to the guide portion 55 even if the insertion portion 15 and the guide portion 55 buckle at their midway portions. And if the insertion portion 15 is pushed out in this way, buckling positions of the insertion portion 15 and the guide portion 55 can be relatively displaced, thereby enabling the recovery from the buckled state if it is a slight buckling or the like.

It is noted that as the diameter of each portion when forming each tapered surface, for example, the outer diameter of the distal end of the insertion portion 15 can be set to 15 mm, and the outer diameter of the proximal end to 10 mm, as well as the inner diameter of the distal end of the guide portion 55 being set to 16 mm, and the inner diameter of the proximal end to 11 mm.

By the way, in general, various functional sections including the image pickup unit 34 are provided close together in the distal end portion 12 of the insertion portion 15 of the endoscope 2. Therefore, it is desirable that the outer diameter of the distal end portion 12 is formed to be as large as possible. On the other hand, the insertion diameter as the whole becomes large in the endoscope apparatus 1 in which the insertion aid 50 is mounted on the outer circumference side of the insertion portion 15. Accordingly, for example, as shown in Fig. 18, by arranging that the outer diameter of the distal end portion 12 of the insertion portion 15 corresponds to the outer diameter of the guide portion 55, and the outer diameters of the bending portion 13 and the flexible pipe portion 14 are smaller than the outer diameter of the distal end portion 12 so as to be insertable into the guide portion 55, it becomes possible to mount the insertion aid 50 to the insertion portion 15 without increasing the insertion diameter as the whole. It is noted that as it is clear from the insertion procedure shown in Figs. 8 to 14, since there is no need of protruding the distal end of the guide portion 55 further forward than the distal end of the insertion portion 15, it is possible to realize a good insertability into a subject, even in such configuration as described above.

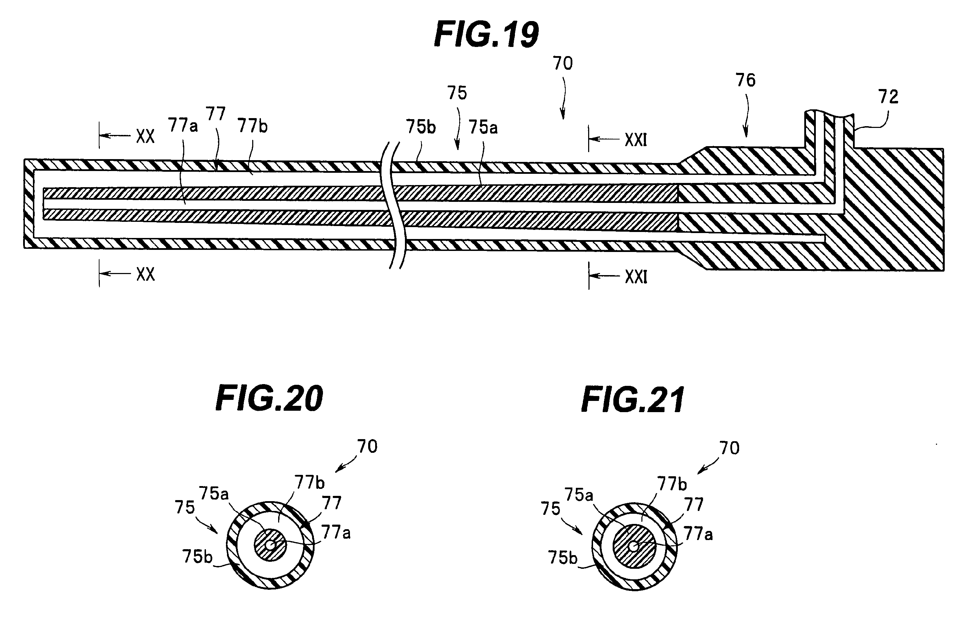

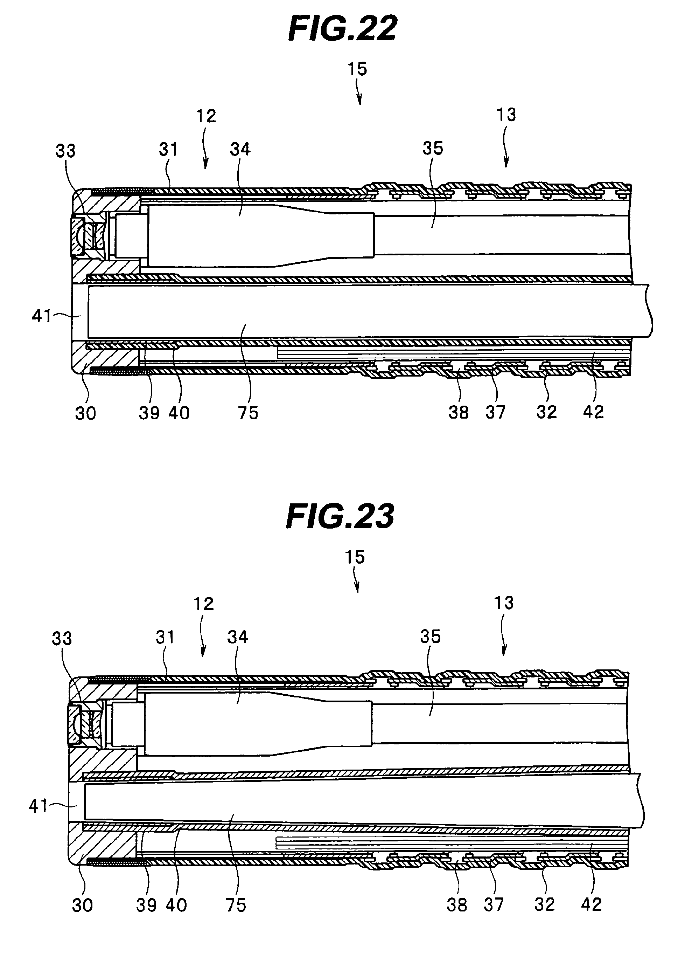

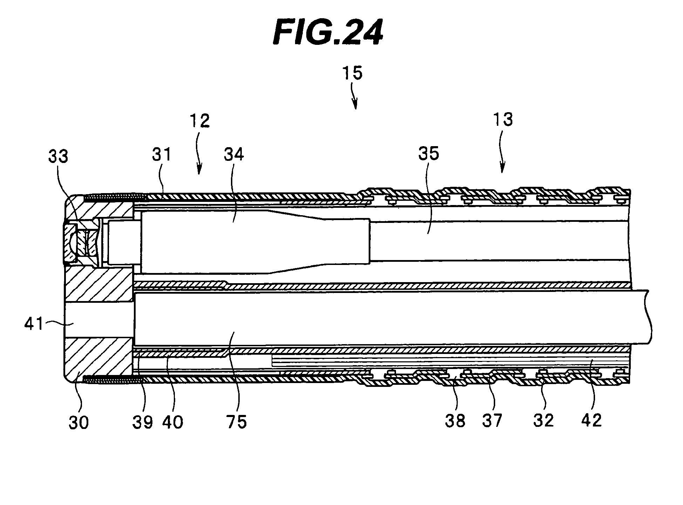

Next, Figs. 19 to 24 relate to a second embodiment of the present invention, in which Fig. 19 is a principal-part cross-sectional view of an insertion aid, Fig. 20 is a XX-XX cross-sectional view of Fig. 19, Fig. 21 is a XXI-XXI cross-sectional view of Fig. 19, Fig. 22 is a cross-sectional view showing a principal part of the insertion portion with the insertion aid being inserted thereinto, and Figs. 23 and 24 are principal-part cross-sectional views showing variants of the insertion portion and the insertion aid. It is noted that the present embodiment mainly differs from the first embodiment in which the insertion aid 50 is arranged along the outside of the insertion portion 15, in that the insertion aid 50 is arranged along the inside of the insertion portion 15. Further, like components are given the same reference symbols and the description thereof is omitted.

As shown in Figs. 19 to 22, an insertion aid 70 of the present embodiment is made up of a rod-shaped member which is to be inserted into an insertion channel 41 of the insertion portion 15. To be specific, the insertion aid 70 is configured to include a guide portion 75 which is long and takes on a substantially cylindrical shape, and a grip portion 76 which is provided in connection with the base portion of the guide portion 75, so that the guide portion 75 is insertable into the insertion channel 41 of the endoscope 2.

A principal part of the guide portion 75 is configured such that the first and second tubes 75a and 75b which extend in the longitudinal direction are arranged on concentric circles.

Among these first and second tubes 75a and 75b, the second tube 75b which is located at the outer circumference is made up of a tube which is made of resin having flexibility. It is noted that the outer diameter of the second tube 75b is set to be slightly smaller than the inner diameter of the insertion channel 41 of the endoscope 2 so that the guide portion 75 is relatively movable in the insertion channel 41 along the longitudinal direction.

The first tube 75a is made up of, for example, a tube which utilizes a strength variable member whose strength changes according to temperature. In the present embodiment, as the strength variable member, a thermosoftening polymer which softens at the high-temperature side and hardens at the low-temperature side can be used as in the first embodiment.

The outer diameter of the first tube 75a is prescribed to be smaller than the inner diameter of the second tube 75b. As a result of this, an air gap 77b is formed between the outer circumferential face of the first tube and the inner circumferential face of the second tube 75b. The air gap 77b is made in communication with the air gap 77a in the first tube 75a so that a series of flow path 77 is formed inside the guide portion 75.

Each end portion of the flow path 77 is connected to the control apparatus 51 via a fluid supply duct 72 which extends from a grip portion 76. This enables the control apparatus 51 to circulate control water whose temperature is adjusted at a predetermined temperature as control fluid in the flow path 77, thereby controlling the heating or cooling of the first tube 75a through the control fluid that flows in the flow path 77. Thus, when the entire first tube 75a is softened by the heating through the control fluid, the guide portion 75 turns into a first state in which it has a small shape retaining force and is flexible. On the other hand, when the entire first tube 75a is hardened by the cooling through the control fluid, the guide portion 75 turns into a second state in which it has a large shape retaining force and its flexibility is limited.

In the present embodiment, the flexural rigidity of the guide portion 75 is prescribed based on, for example, the relation with the flexural rigidity of the insertion portion 15 of the endoscope 2. That is, the flexural rigidity of the guide portion 75 is prescribed to be relatively lower than the flexural rigidity of the insertion portion 15 when the guide portion 75 is the first state, and relatively higher than the flexural rigidity of the insertion portion 15 when the guide portion 75 is in the second state. In this case, it is particularly preferable that the flexural rigidity of the guide portion 75 when it is in the second state is not less than three times the flexural rigidity of the insertion portion 15. Moreover, it is desirable that the flexural rigidity of the guide portion 75 when it is in the second state is not less than five times the flexural rigidity of the guide portion 75 when in the first state.

In addition, a gradient is prescribed in the flexural rigidity of the guide portion 75 itself such that the flexural rigidity of the proximal end side is relatively larger than the flexural rigidity of the distal end side. Such a gradient of flexural rigidity is realized by, for example, as shown in Figs.19 to 21, providing a gradient in wall thickness in the first tube 75a such that the wall thickness of the proximal end side is relatively larger than the wall thickness of the distal end side. That is, by making up the first tube 75a with a tapered pipe having a gradient in its wall thickness, a gradient of flexural rigidity is prescribed in the guide portion 75 such that the flexural rigidity of the proximal end side is relatively larger than the flexural rigidity of the distal end side in either of the first state or the second state.

According to such embodiment as described above, substantially similar working effects as those of the first embodiment described above can be achieved.

Here, as the configuration to provide a gradient in the flexural rigidity of the guide portion 75, a configuration which combines multiple kinds of thermosoftening polymers having different glass transition temperatures may be adopted as in the first embodiment described above.

Moreover, as the countermeasure to enable the continuation of the insertion operation even in an accidental case in which the insertion portion 15 and the guide portion 75 buckle at their midway portions when being inserted into the subject, for example, as shown in Fig. 23, it is possible to form the inner circumferential surface of the channel tube 40 with a tapered surface in which the inner diameter of the distal end side is relatively smaller than the inner diameter of the proximal end side, and to form the outer circumferential face of the guide portion 75 with a tapered surface in which the outer diameter of the distal end side is relatively smaller than the outer diameter of the proximal end side.

Forming each face with such a tapered surface makes it easy to push out the insertion portion 15 relatively with respect to the guide portion 75 even if the insertion portion 15 and the guide portion 75 buckle at their midway portions. And if the insertion portion 15 is pushed out in this way, buckling positions of the insertion portion 15 and the guide portion 75 can be relatively displaced, thereby enabling the recovery from the buckled state if it is a slight buckling or the like.

It is noted that as the diameter of each portion when forming each tapered surface, for example, the inner diameter of the distal end of the channel tube 40 can be set to 2.2 mm, and the inner diameter of the proximal end can be set to 3.7 mm, as well as the outer diameter of the distal end of the guide portion 75 being set to 2 mm, and the outer diameter of the proximal end being set to 3.5 mm.

By the way, in general, various functional sections including the image pickup unit 34 are provided close together in the distal end portion 12 of the insertion portion 15 of the endoscope 2. Therefore, it is desirable that the inner diameter of the opening of the insertion channel 41 which opens to the distal-end rigid portion 30 is formed to be as small as possible. On the other hand, in order to secure a predetermined flexural rigidity of the guide portion 75 to be inserted into the insertion channel 41, it is necessary to set the outer diameter of the guide portion 75 to be large to some degree. Accordingly, for example, as shown in Fig. 24, by arranging that only the inner diameter of the opening of the insertion channel 41 which opens to the distal-end rigid portion 30 is smaller than the outer diameter of the guide portion 75, and prescribing the inner diameter of the channel tube 40 that follows thereafter based on the outer diameter of the guide portion 75, it becomes possible to mount the insertion aid 70 to the insertion portion 15 without increasing the diameter of the insertion portion 15. It is noted that since there is no need of protruding the distal end of the guide portion 75 further forward than the distal end of the insertion portion 15, it is possible to realize a good insertability into a subject even in such configuration as described above.

It is noted that each embodiment described above is configured such that a gradient is provided in the flexural rigidity of the guide portion 55 or 75, in addition to such configurations, for example, a gradient may be provided for the insertion portion 15 as well such that the flexural rigidity becomes higher from the distal end side to the proximal end side.

## Claims

1. An insertion aid (50; 70), comprising:
a long guide portion (55; 75) arranged to be relatively movable in a longitudinal direction along an insertion portion (15) of an endoscope (2) to be inserted into a subject, the guide portion (55; 75) being able to undergo a state change between a first state in which flexural rigidity is low and shape retaining force is small, and a second state in which flexural rigidity is high and shape retaining force is large, wherein
the guide portion (55; 75) includes a strength variable member (55b; 75a) which is arranged to extend along a longitudinal direction of the guide portion (55; 75) and whose strength varies according to temperature,
**characterized in that**
a gradient in flexural rigidity is provided such that a flexural rigidity of a proximal end side of the guide portion (55; 75) is relatively higher than the flexural rigidity of a distal end side of the guide portion (55; 75), wherein a glass transition temperature at which a proximal end side of the strength variable member (55b; 75a) starts hardening is adapted to be relatively lower than the glass transition temperature of a distal end side of the strength variable member (55b; 75a).

2. The insertion aid (50; 70) according to claim 1, wherein
a wall thickness of the proximal end side of the strength variable member (55b; 75a) is set to be relatively larger than a wall thickness of the distal end side of the strength variable member (55b; 75a) in the guide portion (55; 75).

3. The insertion aid (50; 70) according to claim 1, wherein
the guide portion (55; 75) includes a portion made up of a plurality of tubes that lie on concentric circles and extend in a longitudinal direction of the guide portion (55; 75), and
the strength variable member (55b; 75a) is made up of any of the plurality of tubes.

4. An endoscope apparatus (1), comprising:
an endoscope (2) including an insertion portion (15) to be inserted into a subject, and
an insertion aid (50; 70) according to claim 1, wherein
the flexural rigidity of the insertion portion (15) is prescribed to be relative higher than the flexural rigidity of the guide portion (55; 75) when in the first state, and relatively lower than the flexural rigidity of the guide portion (55; 75) when in the second state.

5. The endoscope apparatus (1) according to claim 4, wherein
the insertion portion (15) has a shape retaining capability for retaining the shape while being bent.

6. The endoscope apparatus (1) according to claim 4, wherein
the guide portion (55; 75) is a cylindrical member which is disposed at an outer circumference of the insertion portion (15),
an outer circumferential face of the insertion portion (15) has a tapered surface in which an outer diameter of the distal end side is relatively larger than an outer diameter of the proximal end side, and
an inner circumferential face of the guide portion (55; 75) has a tapered surface in which an inner diameter of the distal end side is relatively larger than an inner diameter of the proximal end side.

7. The endoscope apparatus (1) according to claim 4, wherein
the guide portion (55; 75) is a rod-shaped member which is inserted into an insertion channel (41) formed inside the insertion portion (15),
an inner circumferential face of the insertion channel (41) has a tapered surface in which an inner diameter of the distal end side is relatively smaller than an inner diameter of the proximal end side, and
an outer circumferential face of the guide portion (55; 75) has a tapered surface in which an outer diameter of the distal end side is relatively smaller than an outer diameter of the proximal end side.

## Patentansprüche

1. Einführungshilfe (50, 70) aufweisend:
ein langes Führungsteil (55; 75), das zur relativen Bewegung in einer Längsrichtung entlang eines Einführungsteils (15) eines Endoskops (2), welches in ein Subjekt eingeführt wird, angeordnet ist, wobei das Führungsteil (55; 75) dazu in der Lage ist, einen Zustandswechsel zwischen einem ersten Zustand, in dem die Biegesteifigkeit gering und die Formhaltekraft klein ist, und einem zweiten Zustand, in dem die Biegesteifigkeit hoch und die Formhaltekraft groß ist, zu durchlaufen, wobei
das Führungsteil (55; 75) ein stärkeveränderbares Element (55b; 75a) enthält, das zum Erstrecken entlang einer Längsrichtung des Führungsteils (55; 75) angeordnet ist und dessen Stärke sich gemäß der Temperatur ändert,
**dadurch gekennzeichnet, dass**
ein Gradient an Biegesteifigkeit derart bereitgestellt ist, dass eine Biegesteifigkeit einer proximalen Endseite des Führungsteils (55, 75) relativ größer als die Biegesteifigkeit einer distalen Endseite des Führungsteils (55; 75) ist, wobei eine Glasübergangstemperatur, bei der sich eine proximale Endseite des stärkenveränderbaren Elements (55b; 75a) zu härten beginnt, dazu angepasst ist, relativ keiner als eine Glasübergangstemperatur einer distalen Endseite des stärkeveränderbaren Elements (55b; 75a) zu sein.

2. Einführungshilfe (50; 70) gemäß Anspruch 1, bei der
eine Wanddicke der proximalen Endseite des stärkeveränderbaren Elements (55b; 75a) derart festgelegt ist, dass sie relativ größer als eine Wanddicke der distalen Endseite des stärkeveränderbaren Elements (55b; 75b) in dem Führungsteil (55; 75) ist.

3. Einführungshilfe nach Anspruch 1, bei der
das Führungsteil (55; 75) ein Teil enthält, das aus einer Mehrzahl von Röhren hergestellt ist, die auf konzentrischen Kreisen liegen und sich in eine Längsrichtung des Führungsteils (55; 75) erstrecken; und
das stärkeveränderbare Element (55b; 75a) aus einer der Mehrzahl von Röhren hergestellt ist.

4. Endoskopvorrichtung (1) aufweisend:
ein Endoskop (2) enthaltend ein Einführungsteil (15), das in ein Subjekt einführbar ist, und
eine Einführungshilfe (50; 70) nach Anspruch 1, wobei
die Biegesteifigkeit des Einführungsteils (15) relativ größer als die Biegesteifigkeit des Führungsteils (55; 75) in dem ersten Zustand und relativ kleiner als die Biegesteifigkeit des Führungsteils (55; 75) in dem zweiten Zustand vorgeschrieben ist.

5. Endoskopvorrichtung (1) nach Anspruch 4, bei der
das Einführungsteil (15) eine Formhaltefähigkeit zum Beibehalten der Form während des Biegens aufweist.

6. Endoskopvorrichtung (1) nach Anspruch 4, bei der
das Führungsteil (55, 75) ein zylindrisches Element ist, das an einem äußeren Umfang des Einführungsteils (15) angeordnet ist,
eine äußere Umfangsfläche des Einführungsteils (15) eine kegelförmige Oberfläche aufweist, in der ein äußerer Durchmesser der distalen Endseite relativ größer als ein äußerer Durchmesser der proximalen Endseite ist, und
eine innere Umfangsfläche des Führungsteils (55, 75) eine Kegelfläche aufweist, in der ein inneren Durchmesser der distalen Endseite relativ größer als ein innerer Durchmesser der proximalen Endseite ist.

7. Endoskopvorrichtung (1) nach Anspruch 4, bei der
das Führungsteil (55; 75) ein stabförmiges Element ist, das in einen innerhalb des Einführungsteils (15) gebildeten Einführungskanal (41) eingeführt ist,
eine innere Umfangsfläche des Einführungskanals (41) eine kegelförmige Fläche aufweist, in der ein innerer Durchmesser der distalen Endseite relativ kleiner als ein innerer Durchmesser der proximalen Endseite ist, und
eine äußere Umfangsfläche des Führungsteils (55; 75) eine kegelförmige Fläche aufweist, in der ein äußerer Durchmesser der distalen Endseite relativ kleiner als einer äußerer Durchmesser der proximalen Endseite ist.

## Revendications

1. Aide à l'insertion (50 ; 70), comprenant :
une partie de guidage longue (55 ; 75) agencée pour être relativement mobile dans une direction longitudinale le long d'une partie d'insertion (15) d'un endoscope (2) destiné à être inséré dans un sujet, la partie du guidage (55 ; 75) étant capable de subir un changement d'état entre un premier état dans lequel la rigidité à la flexion est faible et une force de rétention de forme est faible, et un deuxième état dans lequel la rigidité à la flexion est élevée et une force de rétention de forme est importante, dans laquelle
la partie de guidage (55 ; 75) comprend un élément à résistance variable (55b ; 75a) qui est agencé pour s'étendre le long d'une direction longitudinale de la partie de guidage (55 ; 75) et dont la résistance varie selon la température,
**caractérisée en ce que**
un gradient dans la rigidité à la flexion est prévu d'une manière telle qu'une rigidité à la flexion d'un côté d'extrémité proximale de la partie de guidage (55 ; 75) est relativement plus élevée que la rigidité à la flexion d'un côté d'extrémité distale de la partie du guidage (55 ; 75), dans laquelle une température de transition vitreuse à laquelle un côté d'extrémité proximale de l'élément à résistance variable (55b ; 75a) commence à durcir est adaptée pour être relativement inférieure à la température de transition vitreuse d'un côté d'extrémité distale de l'élément à résistance variable (55b ; 75a).

2. Aide à l'insertion (50 ; 70) selon la revendication 1, dans laquelle
une épaisseur de paroi du côté d'extrémité proximale de l'élément à résistance variable (55b ; 75a) est établie pour être relativement supérieure à une épaisseur de paroi du côté d'extrémité distale de l'élément à résistance variable (55b ; 75a) dans la partie du guidage (55 ; 75).

3. Aide à l'insertion (50 ; 70) selon la revendication 1, dans laquelle
la partie du guidage (55 ; 75) comprend une partie constituée d'une pluralité de tubes qui reposent en cercles concentriques et s'étendent dans une direction longitudinale de la partie du guidage (55 ; 75), et
l'élément à résistance variable (55b ; 75a) est constitué de l'un quelconque parmi la pluralité de tubes.

4. Appareil (1) d'endoscope, comprenant :
un endoscope (2) comprenant une partie d'insertion (15) destinée à être insérée dans un sujet, et
une aide à l'insertion (50 ; 70) selon la revendication 1, dans laquelle
la rigidité à la flexion de la partie d'insertion (15) est prescrite pour être relativement supérieure à la rigidité à la flexion de la partie du guidage (55 ; 75) lorsqu'elle se trouve dans le premier état, et relativement inférieure à la rigidité à la flexion de la partie de guidage (55 ; 75) lorsqu'elle se trouve dans le deuxième état.

5. Appareil (1) d'endoscope selon la revendication 4, dans lequel
la partie d'insertion (15) possède une capacité de rétention de forme destinée à retenir la forme tout en étant courbée.

6. Appareil (1) d'endoscope selon la revendication 4, dans lequel
la partie de guidage (55 ; 75) est un élément cylindrique qui est disposé au niveau d'une circonférence externe de la partie d'insertion (15),
une face circonférentielle externe de la partie d'insertion (15) présente une surface conique dans laquelle un diamètre externe du côté d'extrémité distale est relativement plus grand qu'un diamètre externe du côté d'extrémité proximale, et
une face circonférentielle interne de la partie de guidage (55 ; 75) présente une surface conique dans laquelle un diamètre interne du côté d'extrémité distale est relativement plus grand qu'un diamètre interne du côté d'extrémité proximale.

7. Appareil (1) d'endoscope selon la revendication 4, dans lequel
la partie de guidage (55 ; 75) est un élément en forme de tige qui est inséré dans un canal d'insertion (41) formé à l'intérieur de la partie d'insertion (15),
une face circonférentielle interne du canal d'insertion (41) présente une surface conique dans laquelle un diamètre interne du côté d'extrémité distale est relativement plus petit qu'un diamètre interne du côté d'extrémité proximale, et
une face circonférentielle externe de la partie de guidage (55 ; 75) présente une surface conique dans laquelle un diamètre externe du côté d'extrémité distale est relativement plus petit qu'un diamètre externe du côté d'extrémité proximale.
